# EUROPEAN PATENT APPLICATION

(11) **EP 2 308 299 A1**
(43) Date of publication of application: **13.04.2011**
(21) Application number: 10185859.5
(22) Date of filing: 12.01.2006
(51) Int. Cl.: A01N 43/40, A01P 1/00, A61K 8/49, A61K 8/33, A61K 8/34, A61Q 19/10, A61K 8/39, A61K 8/41, A01N 31/02, A01N 33/12

(54) **Disinfectant composition including glycerol monoalkyl ethers and bispyridiniumalkanes and use as skin antiseptic**

(30) Priority: 19.01.2005 DE 102005002644
(62) Divisional of application: 06100301.8
(71) Applicant: Air Liquide Santé (International), 75007 Paris (FR); SCHÜLKE & MAYR GMBH, 22851 Norderstedt (DE)
(72) Inventor: Beilfuss, Wolfgang, 22339 Hamburg (DE); Behrends, Sabine, 25482 Appen (DE); Goroncy-Bermes, Peter, 22145 Hamburg (DE); Puchstein, Burghard, 20257 Hamburg (DE)
(74) Representative: Beroud, Amandine

(57) **Abstract**

The invention relates to a disinfectant composition which includes
a) 1-(2-ethylexyl) glycerol ether
b) octenidine dihydrochloride, and
c) quaternary ammonium compound.

## Description

The present invention relates to disinfectant compositions which are preferably employed for hygienic hand disinfection and disinfectant handwashing. The compositions are suitable in particular as skin antiseptic.

The aim of hand disinfection, hand decontamination and skin antisepsis is to prevent transmission of microorganisms and viruses or to suppress their unwanted introduction into endangered regions of the body or more sensitive regions. Compositions for hygienic hand disinfection and for disinfectant handwashing must satisfy certain efficacy requirements, some of which are defined in standards. Various methods are possible for treating the hands after contamination.

Hygienic hand disinfection complying with EN 1500 as rubbing method without addition of water causes the death or inactivation of the transient microorganisms on the hands, without any risk of microbes being disseminated in the surroundings and without any risk of recontamination of the hands by microorganisms possibly present in the water. Disinfectant handwashing complying with EN 1499 with a microbicidal composition using tap water is likewise directed against transient microorganisms without precluding their dissemination in the surroundings. It serves in particular to reduce microbes during the washing procedure, but cannot replace hand disinfection. In disinfectant handwashing, the composition is rubbed in undiluted and foamed with a little water, and the hands are cleaned and thoroughly rinsed with water.

Compositions for disinfectant handwashing and hygienic hand disinfection must be effective after acting for short times (e.g. 30 seconds or 1 minute). In the efficacy tests, it is important that there is a good effect (RF > 3 orders of magnitude) after acting for these short times. For toxicological reasons it is additionally necessary for, besides adequate efficacy, in particular the compatibility with human skin to be ensured.

Commercially available compositions for disinfectant handwashing are usually alcohol- or surfactant-containing liquid soaps and wash lotions ready for use with further biocidal agents added. Known compositions comprise as biocidal agents for example short-chain alcohols and as excipients superfatting agents, moisturizers and fragrances to improve the skin compatibility and acceptance. To enhance the efficacy and to achieve a residual effect intended to prevent the number of microbes on the hands increasing again, the known compositions often also comprise additional agents such as biguanides (e.g. chlorhexidine), quaternary ammonium compounds (e.g. benzalkonium chloride), phenol derivatives (e.g. ortho-phenylphenol) or carboxylic acids. Thus, for example, a known aqueous composition for hand disinfection includes 0.1% by weight octenidine dihydrochloride and 2% by weight phenoxyethanol and as excipients sodium gluconate, glycerol and cocamidopropylbetaine.

The known compositions for disinfectant handwashing have a number of disadvantages. Thus, some products show an efficacy which is not always satisfactory, or the desired efficacy is achieved only after acting for a lengthy time. Some compositions additionally have insufficient skin compatibility. Thus, products based on chlorhexidine are reported to be prone to skin incompatibility, it being suggested for example that there is partial release of chloroaniline.

An additional factor is that agents with organically bound halogen such as chlorhexidine have only conditional environmental compatibility. Chlorhexidine is moreover sufficiently effective only with a comparatively high concentration of the agent (e.g. 2% by weight) in wash products and may lead to discolorations on contaminated surfaces. Poly(hexamethylenebiguanide) hydrochloride is a polymeric biguanide salt whose structure is not exactly defined. For this reason, no medicinal product with this agent yet has marketing authorization in Germany. The polymeric biguanide may be employed only in a concentration of up to 0.3% by weight in cosmetic compositions.

An additional factor is that known compositions have a comparatively high viscosity and/or a comparatively high surface tension. Such compositions are merely in principle less suitable for hand disinfection because it is indispensable for hand disinfectants to combat microbes on the skin as completely as possible and thus to wet the skin as completely as possible.

Hence there is a need for compositions with good efficacy and, at the same time, good skin compatibility and improved (reduced) surface tension compared with known compositions. In particular, it was an object of the invention to provide a skin antiseptic which is toxicologically acceptable and compatible with skin and has a good microbicidal efficacy.

It has now surprisingly been found that the problems of the prior art can be overcome by a composition which includes a)1-(2-ethylexyl) glycerol ether, b) octenidine dihydrochloride, c) quaternary ammonium compound.
The compositions according to the invention have an excellent effect with, at the same time, good skin compatibility and stability and a markedly reduced surface tension. They show a distinctly improved efficacy compared with known compositions. Moreover, synergistic increases in effect are observed in some cases. The often marked inhibition of the efficacy of octenidine dihydrochloride and 1-(2-ethylexyl) glycerol ether, which is observed with numerous surfactants, does not occur with the quaternary ammonium compound. The compositions can be employed as products for personal hygiene and as medical wash products, high-value, soap-free wash products for all hand, skin and body washing and as bath additive, and for disinfection or decontamination of animate (e.g. skin, hands, mucous membrane, wounds) and inanimate surfaces (e.g. apparatuses, instruments, endoscopes).

Compositions preferred in this connection are those free of aromatic alcohols such as 2-phenoxyethanol, benzyl alcohol, phenethyl alcohol, 1-phenoxypropan-2-ol, 3-(4-chlorophenoxy)-1,2-propanediol, chlorobutanol, 2,4-dichlorobenzyl alcohol or mixtures thereof. Preferred compositions are moreover those including no salts selected from the salts of benzoic acid, propionic acid, salicylic acid, sorbic acid, 4-hydroxybenzoic acid, dehydroacetic acid and 10-undecylenic acid and/or any of the free acids mentioned.

### 1-(2-ethylexyl) glycerol ether

The content of 1-(2-ethylexyl) glycerol ether is generally in the range from 0.03 to 5% by weight, preferably 0.05 to 3% by weight, more preferably 0.1 to 1% by weight, such as 0.15 to 0.5% by weight, for example about 0.25% by weight.

### octenidine dihydrochloride

In a preferred embodiment, the content of octenidine dihydrochloride is in the range from 0.01 to 2% by weight, more preferably 0.02 to 1.5% by weight, in particular 0.04 to 0.6% by weight, such as 0.06 to 0.2% by weight, for example about 0.1% by weight.
Moreover, it is possible to employ a polyol and/or a surfactant in the disinfectant composition.

### Polyol

All polyols can be employed according to the invention. An embodiment preferred in this connection is one where the polyol has 3 or more hydroxy groups. Preference is further given to an embodiment where the composition comprises one or more polyols, with the optional additional presence of one or more surfactants c1) and/or c2).

Examples of suitable polyols are 1,2-propylene glycol, glycerol, erythritol, 1,2,6-hexanetriol, inositol, lactitol, maltitol, mannitol, methylpropanediol, phytantriol, polyglycerols, sorbitol and xylitol, with particular preference for glycerol.

In a preferred embodiment, the content of polyol is in the range from 0.05 to 10% by weight, more preferably 0.2 to 7% by weight, in particular 0.4 to 5% by weight, such as, for example, about 0.4% by weight or in the range from 1.5 to 4% by weight, for example about 2.4% by weight.

### Nonionic surfactant

It is possible to employ as nonionic surfactant according to the invention all suitable nonionic surfactants, preferably using (fatty) alcohol ethoxylates, alkyl polyglycosides (especially alkyl polyglucosides), amine oxides, ethylene oxide/propylene oxide block copolymers, amphoteric surfactants and ether carboxylic acids, with particular preference for fatty alcohol ethoxylates.

The alcohol polyalkoxylates include fatty alcohol alkoxylates, e.g. isodecyl ethoxylates with various proportions of ethylene oxide, isotridecyl ethoxylates, polyethylene glycol ethers of stearyl, lauryl and cetyl and oleyl alcohol. It is possible in this connection for the alcohols to have been alkoxylated with ethylene oxide, propylene oxide or any mixtures of ethylene oxide and propylene oxide. Alcohol polyalkoxylates are known inter alia under the names Lutensol^{®}, Marlipal^{®}, Marlox^{®}, Brij^{®} and Plurafac^{®}. Lauryl alcohol ethoxylates are particularly preferred as nonionic surfactant.

Also employed as nonionic surfactants are the sorbitan esters which are in the form of oleates, stearates, laurates and palmitates and are referred to as polysorbates (e.g. Tween^{®}). It is additionally possible for the nonionic surfactant to be an alkyl glycoside such as an alkyl glucoside (i.e. an alkyl glycoside of glucose), more preferably a C₈- to C₂₀-alkylpolyglucose, in particular a C₈- to C₁₆-alkylpolyglucose, with preference for a lauryl polyglucose, a decyl polyglucose or a mixture thereof. The C-chain length in the case of cocoyl polyglucose is from 8 to 16 C atoms, in the case of lauryl polyglucose is from 12 to 16 C atoms and in the case of decyl polyglucose is likewise from 8 to 16 C atoms. The nonionic surfactant which is particularly preferably employed is the lauryl ethoxylate marketed under the name Brij^{®} 35P (laureth-35, lauryl alcohol with 35 EO units).

In a preferred embodiment, the amount of the nonionic surfactant is from 0.005 to 1% by weight, more preferably 0.01 to 0.1% by weight, such as 0.015 to 0.05% by weight, for example about 0.02% by weight.

### Quaternary ammonium compound

It is possible in principle to employ according to the invention all suitable quaternary ammonium compounds. The quaternary ammonium compound is preferably selected from betaines and dimethylammonium salts.

Quaternary ammonium salts employed according to the invention are represented by the formula [R¹R²R³(CH₃)N]⁺[X]⁻, where R¹ to R³ may be identical or different and are selected from C₁- to C₃₀-alkyl, aralkyl, -alkenyl and mixed groups which may have one or more atoms selected from O, S, N and P, where R¹ to R³ are for example C₈- to C₁₈-alkyl, benzyl or methyl, preferably C₉- to C₁₈-alkyl, benzyl or methyl, such as C₁₆-alkyl, benzyl or methyl. X is an anion (of an inorganic or organic acid). It is possible in this connection for both anion and cation of the quaternary ammonium salt to be multiply charged ions, resulting in a stoichiometry [A⁽ⁿ⁺⁾]ₘ[K^{(m+)}]ₙ.

Suitable quaternary ammonium salts according to the invention are all quaternary ammonium salts of the abovementioned formula which are known in the art, as disclosed for example in WO 00/63337, which is incorporated herein by reference. However, dialkyldimethylammonium salts are preferably employed, for example dialkyldimethylammonium chlorides whose alkyl chains are selected independently of one another from C₈- to C₁₈-alkyl, preferably C₉- to C₁₈-alkyl, such as C₁₆-alkyl. One of the methyl groups in the dialkyldimethylammonium salts may be an alkoxylated, for example ethoxylated, hydroxymethyl group.

Quaternary ammonium salts which are preferably employed according to the invention are compounds of the formulae [R¹N (CH₃)₃]⁺[X]⁻, [R¹R²N(CH₃)₂]⁺[X]⁻ and [R¹R²R³(CH₃)_{N}]⁺[X]⁻, where R¹ to R³ are selected independently of one another from C₈- to C₁₈-alkyl and -(CH₂-CHR⁴O)ₙ-R⁵, where n is a number from 1 to 20, preferably 1 to 5, and R⁴ and R⁵, which may be identical or different, are H and/or C₁- to C₄-alkyl, preferably H.

Examples of anions and classes of anions of the quaternary ammonium salts employed according to the invention are hydroxide, sulphate, hydrogensulphate, methosulphate, ethosulphate, lauryl sulphate, lauryl ether sulphate, cellulose sulphate, sulphamate, halide (fluoride, chloride, bromide, iodide), nitrite, nitrate, carbonate, hydrogencarbonate, phosphate, alkyl phosphate, metaphosphate, polyphosphate, thiocyanate, carboxylic salt such as benzoate, lactate, acetate, propionate, citrate, succinate, glutarate, adipate, toluenesulphonate (tosylate) and salicylate. Particularly preferred anions are chloride and propionate.

The quaternary ammonium salts which are particularly preferably employed are mecetronium etilsulfate (hexadecyl(ethyl)dimethylammonium ethyl sulphate) and benzalkonium chloride.

All suitable betaines can be employed as betaine. Cocamidopropylbetaine is particularly preferred.

A preferred amount of quaternary ammonium compound is in the range from 0.01 to 5% by weight. The amount of betaine is preferably in the range from 0.03 to 5% by weight, more preferably 0.05 to 3% by weight, in particular 0.1 to 1% by weight, such as 0.15 to 0.5% by weight, and is for example about 0.3% by weight. The amount of quaternary ammonium salt (for example mecetronium etilsulfate or benzalkonium chloride) is preferably from 0.01 to 5% by weight, more preferably 0.02 to 2.5% by weight, in particular 0.04 to 0.8% by weight, such as 0.05 to 0.4% by weight.

Compositions which are additionally preferred are those additionally including from 0.05 to 0.5% by weight, more preferably 0.1 to 0.3% by weight of skin care additives such as allantoin or sodium gluconate.

The composition of the invention is preferably in the form of an aqueous composition and comprises from 5 to 99.5% by weight, more preferably 30 to 99% by weight, in particular 50 to 98.5% by weight, such as 80 to 98% by weight, 90 to 97.5% by weight, for example 96 to 97% by weight, based on the composition.

A particularly preferred composition includes:
a) 0.15 to 5% by weight 1-(2-ethylhexyl)glycerol ether,
b) 0.06 to 0.2% by weight octenidine dihydrochloride and
c) 1.5 to 4% by weight glycerol
   and where appropriate
   c1) 0.015 to 0.05% by weight nonionic surfactant,
   c2) 0.15 to 0.5% by weight quaternary ammonium compound, preferably cocamidopropylbetaine, benzalkonium chloride or mecetronium etilsulfate and/or
d) 0.2 to 0.6% by weight sodium gluconate.

The composition may additionally comprise functional additives such as colorants, perfume, buffers, electrolytes and moisturizing factors. The preferred pH of the composition is from 4 to 8, preferably 5 to 6.

The invention further relates to the use of the compositions for hygienic hand disinfection or for disinfectant handwashing, in particular as skin antiseptic. The compositions according to the invention are suitable for hygienic and surgical hand disinfection, hygienic handwashing, hand decontamination, skin decontamination, personal hygiene washing lotion, as antimicrobial washing lotion, for (whole) body washing and care in connection with MRS (methicillin-resistant Staphylococcus aureus) and furthermore for disinfectant handwashing, for hygienic catheter care in patients, as handwashing product such as, for example, as antimicrobial soap, handwashing gel or handwashing lotion. They can be employed advantageously in all sectors with enhanced hygiene requirements in the medical and nonmedical sector, e.g. hospitals, medical practices, old people's and nursing homes, and in the food products and kitchen sectors. Compositions according to the invention may also be formulated as alcoholic and disinfectants, gel compositions, ointment compositions and antimicrobial coatings.

The invention additionally relates to a composition which includes
a) 3-(1-menthoxy)-1,2-propanediol (glycerol monomenthyl ether) and
b) one or more bispyridiniumalkanes
   and where appropriate
c) one or more polyols and/or one or more surfactants selected from the group of c1) nonionic surfactant and c2) quaternary ammonium.

In this last-mentioned embodiment, the presence of component c) is optional. The above remarks concerning preferred glycerol monoalkyl ethers, bispyridiniumalkanes etc. and the amounts employed in each case apply correspondingly. In such a composition according to the invention, the water-solubility of glycerol monomenthyl ether is significantly improved by the addition of the bispyridiniumalkane (especially octenidine dihydrochloride). Such compositions according to the invention can be used for hygienic hand disinfection or for disinfectant handwashing, as skin disinfectant, antiseptic, wound antiseptic, deodorant or oral hygiene product.

It was surprising that an effect is achieved after only a short time with compositions according to the invention containing glycerol monoalkyl ether for hand disinfection. Although glycerol monoalkyl ethers are known, for example from DE 42 40 674 C1, to act as deodorant agents, disinfection is immaterial for deodorants. For this reason, known deodorant agents merely have antimicrobial activity, whereas hand disinfectants must have microbicidal activity. An additional factor is that glycerol monoalkyl ethers on their own have virtually no microbicidal effect, and accordingly are unsuitable as (sole) agents for hand disinfection. It was therefore surprising that glycerol monoalkyl ethers contribute in the compositions according to the invention to the hand-disinfectant effect.

In addition, deodorant compositions which are intended by their nature to remain on the skin for a lengthy period are preferably free of surfactants. Thus, compatibility between deodorant agents and surfactants is immaterial in deodorant compositions. By contrast, it was surprising that compositions according to the invention can be formulated with nonionic surfactants and quaternary ammonium compounds without the efficacy of the bispyridiniumalkanes and glycerol monoalkyl ethers being impaired.

Compositions according to the invention, which are preferably employed as skin antiseptics, medicinal products, antiseptics and wound disinfectants, are, in a preferred embodiment, free of phenoxyethanol and/or phenoxypropanol and are toxicologically acceptable. The compositions according to the invention additionally have the following advantages:
- Good tolerability in combination with good antimicrobial efficacy and excellent short-term effect (acting for a time of, for example, 30 or 60 seconds), and very good effect on Gram-negative bacteria.
- Good efficacy for viruses (enveloped and non-enveloped) and multidrug-resistant microorganisms such as MRSA.
- No need to add a further preservative because they are self-preservative.
- The solubility in water and water-based compositions of glycerol monoalkyl ethers (such as 1-(2-ethylhexyl)glycerol ether) is only limited (solubility in water 0.1% by weight). The presence of bispyridiniumalkane in the compositions according to the invention leads to a significantly improved solubility in water of the glycerol monoalkyl ether.
- Low surface tension/low contact angle and thus exceptionally good wetting effect.

The advantages of the invention are evident in particular from the following examples (data in % by weight).

### Examples

The following compositions were formulated (proportions in % by weight).

| | A | B | C | D | E | F (com par.) | G (compar.) | H (compar.) |
|---|---|---|---|---|---|---|---|---|
| 1-(2-Ethyl-hexyl)glycerol ether | 0.25 | 0.25 | 0.25 | 0.25 | 0.25 | - | - | - |
| Octenidine 2HCl | 0.10 | 0.10 | 0.10 | 0.10 | 0.10 | 0.10 | 0.10 | 0.10 |
| Glycerol | 2.42 | 2.42 | 2.42 | 2.42 | 2.42 | 0.42 | 2.42 | 2.42 |
| Laureth-35 | 0.02 | - | - | - | - | - | - | 0.02 |
| Phenoxy-ethanol | - | - | - | - | - | 2.00 | - | - |
| Mecetronium etilsulfate | - | - | 0.10 | 0.10 - | - | - | - | - |
| Benzalkonium chloride | Benzalkonium - | - | - | 0.20 | - | - | - | - |
| Sodium gluconate | - | - | - | - | 0.40 | 0.40 | - | - |
| Cocamidopropylbetaine | - | - | - | - | 0.30 | 0.30 | - | - |
| Deionized water | ad 100 | ad 100 | ad 100 | ad 100 | ad 100 | ad 100 | ad 100 | ad 100 |

### Investigation of the surface tension and of the contact angle

Determinations of the surface tension and of the contact angle (on glass) were carried out with compositions A, F, G and H. The contact angle was determined by employing a G10 contact angle measuring instrument supplied by A. Krüss Optronik GmbH, Hamburg, Germany. The surface tension was measured by the hanging drop method using a DSA10 instrument and the software from A. Krüss Optronik GmbH, Hamburg, Germany.

| Composition | Surface tension mN/m | Contact angle on glass, ° |
|---|---|---|
| A | 27.01 | 12.9 |
| F (comparative) | 32.08 | 25.8 |
| G (comparative) | 52.16 | 49.5 |
| H (comparative | 45.33 | 51.5 |

### Method for determining the efficacy of the compositions

The efficacy of the compositions according to the invention was tested in the quantitative suspension test. Compare standard methods of the DGHM for testing chemical disinfection methods, J. Gebel, H.-P. Werner, A. Kirsch-Altena, K. Bansemir, mhp Verlag GmbH, Wiesbaden, Germany, method 9.1 (date: 1 September 2001) (quantitative suspension test with bacteria (apart from mycobacteria) and fungi).

**Efficacy tests**

| **S. aureus 50% with stress** | | | | |
|---|---|---|---|---|
| | 0.5 min | 1 min | 3 min | 5 min |
| B | 5.82 | 6.41 | 6.42 | 6.40 |
| C | 5.58 | 6.41 | 6.42 | 6.40 |
| D | 6.43 | 6.41 | 6.42 | 6.40 |
| F (comparative) | 6.43 | 6.41 | 6.42 | 6.40 |
| G (comparative) | 6.13 | 6.41 | 6.42 | 6.40 |
| | | | | |

| **P. aeruginosa 50% with stress** | | | | |
|---|---|---|---|---|
| | 0.5 min | 1 min | 3 min | 5 min |
| B | 6.41 | 6.39 | 6.34 | 6.38 |
| C | 3.51 | 4.77 | 6.34 | 6.38 |
| D | 6.41 | 6.39 | 6.34 | 6.38 |
| F (comparative) | 5.15 | 6.39 | 6.34 | 6.38 |
| G (comparative) | 6.41 | 6.39 | 6.34 | 6.38 |

| **S. aureus 25% with stress** | | | | |
|---|---|---|---|---|
| | 0.5 min | 1 min | 3 min | 5 min |
| B | 4.69 | 5.93 | 6.42 | 6.40 |
| C | 5.10 | 6.11 | 6.42 | 6.40 |
| D | 6.43 | 6.41 | 6.42 | 6.40 |
| F (comparative) | 5.73 | 6.41 | 6.42 | 6.40 |
| G (comparative) | 4.70 | 6.41 | 6.42 | 6.40 |

| **P. aeruginosa 25% with stress** | | | | |
|---|---|---|---|---|
| | 0.5 min | 1 min | 3 min | 5 min |
| B | 4.91 | 5.55 | 6.34 | 6.38 |
| C | 0.00 | 0.00 | 2.67 | 5.08 |
| D | 6.41 | 6.39 | 6.34 | 6.38 |
| F (comparative) | 5.16 | 6.39 | 6.34 | 6.38 |
| G (comparative) | 0.00 | 4.11 | 6.34 | 6.38 |

## Claims

1. Disinfectant composition which includes
a)1-(2-ethylexyl) glycerol ether,
b) octenidine dihydrochloride, and
c) quaternary ammonium compound.

2. Composition according to claim 1, **characterized in that** it includes from 0.03 to 5% by weight 1-(2-ethylexyl) glycerol ether.

3. Composition according to claim 1 or 2, **characterized in that** it includes from 0.01 to 2% by weight octenidine dihydrochloride.

4. Composition according to any of the preceding claims, **characterized in that** the quaternary ammonium compound is selected from betaines and dimethylammonium salts, preferably cocamidopropylbetaine, mecetronium etilsulfate and benzalkonium chloride.

5. Composition according to any of the preceding claims, which includes from 0.01 to 5% by weight quaternary ammonium compound.
